# EUROPEAN PATENT APPLICATION

(11) **EP 3 915 486 A1**
(43) Date of publication of application: **01.12.2021**
(21) Application number: 19911738.3
(22) Date of filing: 01.11.2019
(51) Int. Cl.: A61B 8/08, A61B 8/14, A61B 8/00

(54) **ULTRASONIC IMAGING APPARATUS AND DISPLAY METHOD THEREOF**

(30) Priority: 23.01.2019 KR 20190008605
(71) Applicant: Samsung Medison Co., Ltd., Hongcheon-gun, Gangwon-do 25108 (KR)
(72) Inventor: PARK, Jinki, Seongnam-si, Gyeonggi-do 13530 (KR); LEE, Jinyong, Seongnam-si, Gyeonggi-do 13530 (KR); CHANG, Hyukjae, Seoul 03722 (KR)
(74) Representative: Arnold & Siedsma
(86) International application number: PCT/KR2019/014727
(87) International publication number: WO 2020/153583

(57) **Abstract**

Disclosed are an ultrasound diagnosis apparatus and an ultrasonic image display method. The ultrasound diagnosis apparatus according to a disclosed embodiment may include a display unit, a user interface, a memory for storing one or more instructions, and a processor for executing the one or more instructions to acquire ultrasonic data using a probe that moves on the basis of a scanning line, control the display unit to display the body shape of a user, control the user interface to acquire information on the scanning line, and acquire, on the basis of the body shape and the information on the scanning line, an ultrasonic image volume from the ultrasonic data.

## Description

### [Technical Field]

The present invention relates to an ultrasound diagnosis apparatus and a method thereof. More particularly, the present invention relates to an ultrasound diagnosis apparatus capable of acquiring an ultrasonic image volume from ultrasonic data on the basis of information on a scanning line and a method thereof.

### [Background Art]

Ultrasound diagnosis apparatuses irradiate ultrasonic signals generated by transducers of a probe to an object and receive information about signals reflected from the object, thereby obtaining at least one image of an internal part of the object (e.g., soft tissue or blood flow). In particular, the ultrasound diagnosis apparatuses are used for medical purposes including observation, detection of foreign substances, and assessment of injuries inside an object. Such ultrasound diagnosis apparatuses have advantages in that stability is high, images can be displayed in real time, and the apparatuses are safe because there is no radiation exposure compared to diagnostic apparatuses using X-ray. Accordingly, the ultrasound diagnosis apparatuses have been widely used together with other types of imaging diagnosis apparatuses including computed tomography (CT) apparatuses, magnetic resonance imaging (MRI) apparatuses, and the like.

### [Disclosure]

### [Technical Solution]

According to an embodiment, a method and apparatus for more precisely providing an ultrasonic image volume are provided.

A method of displaying an ultrasonic image according to an embodiment may include steps of acquiring ultrasonic data using a probe that moves on the basis of a scanning line, displaying a body shape of a user, acquiring information on the scanning line through a user interface, and acquiring an ultrasonic image volume from the ultrasonic data on the basis of the body shape and the information on the scanning line.

Further, the method may further include a step of acquiring a height of the user through the user interface, wherein the step of acquiring the ultrasonic image volume from the ultrasonic data may include a step of acquiring an ultrasonic data volume from the ultrasonic data on the basis of the height, the body shape, and the information on the scanning line.

Further, the step of acquiring the ultrasonic image volume from the ultrasonic data may include steps of calculating a length of the scanning line on the basis of the height, the body shape, and the information on the scanning line and acquiring the ultrasonic data volume on the basis of the calculated length.

Further, the body shape may be a shape that illustrates a part of a body of the user.

Further, the step of displaying the body shape of the user may include steps of displaying a plurality of symbols corresponding to a plurality of parts of the body of the user, acquiring a signal for selecting one of the plurality of parts through the user interface, and displaying the selected part as the body shape on the basis of the signal.

Further, the information on the scanning line may be acquired in the form of information indicating a start point and an end point of the scanning line on the displayed body shape.

Further, the scanning line may be approximated in the form of a straight line connecting the start point and the end point.

Further, the scanning line may be approximated in the form of a free curve connecting the start point and the end point on the basis of the body shape of the user.

Further, the information on the scanning line may be acquired in the form of a line indicating the scanning line on the displayed body shape.

Further, the body shape may be provided in at least one type of a front view, a rear view, a cross-sectional view, and a three-dimensional image of a body of the user.

An ultrasound diagnosis apparatus according to an embodiment may include a display unit, a user interface, a memory for storing one or more instructions, and a processor for executing the one or more instructions to acquire ultrasonic data using a probe that moves on the basis of a scanning line, control the display unit to display a body shape of a user, control the user interface to acquire information on the scanning line, and acquire, on the basis of the body shape and the information on the scanning line, an ultrasonic image volume from the ultrasonic data.

Further, the processor may control the user interface to acquire a height of the user, and acquire an ultrasonic data volume from the ultrasonic data on the basis of the height, the body shape, and the information on the scanning line.

Further, the processor may calculate a length of the scanning line on the basis of the height, the body shape, and the information on the scanning line and acquire the ultrasonic data volume on the basis of the calculated length.

Further, the body shape may be a shape that illustrates a part of a body of the user.

The processor may control the display unit to display a plurality of symbols corresponding to a plurality of parts of the body of the user, acquire a signal for selecting one of the plurality of parts through the user interface, and control, on the basis of the signal, the display unit to display the selected part as the body shape.

Further, the information on the scanning line may be acquired in the form of information indicating a start point and an end point of the scanning line on the displayed body shape.

Further, the scanning line may be approximated in the form of a straight line connecting the start point and the end point.

Further, the scanning line may be approximated in the form of a free curve connecting the start point and the end point on the basis of the body shape of the user.

Further, the information on the scanning line may be acquired in the form of a line indicating the scanning line on the displayed body shape.

In a computer-readable recording medium configured to store a computer program code that performs a method of displaying an ultrasonic image, according to an embodiment when being read and executed by a processor, the method of displaying an ultrasonic image may include steps of acquiring ultrasonic data using a probe that moves on the basis of a scanning line, displaying a body shape of a user, acquiring information on the scanning line through a user interface, and acquiring an ultrasonic image volume from the ultrasonic data on the basis of the body shape and the information on the scanning line.

### [Description of Drawings]

The present invention will be more easily understood from the following description taken in conjunction with the accompanying drawings in which reference numerals denote structural elements.
FIG. 1 is a block diagram illustrating a configuration of an ultrasound diagnosis apparatus according to an embodiment.
FIGS. 2A to 2C are views illustrating ultrasound diagnosis apparatuses according to an embodiment.
FIG. 3 is a block diagram illustrating a configuration of an ultrasound diagnosis apparatus according to an embodiment.
FIG. 4 is a flowchart illustrating a method of displaying an ultrasonic image according to an embodiment.
FIGS. 5A and 5B are views for describing a probe according to an embodiment.
FIG. 6 is a view for describing the movement of the probe according to an embodiment.
FIG. 7 is a view for describing a method of acquiring ultrasonic data of an object using the probe of FIG. 5A.
FIG. 8 is a view for describing a method of acquiring ultrasonic data of an object using the probe of FIG. 5B.
FIG. 9 is a view for describing a method of acquiring an ultrasonic image volume on the basis of ultrasonic data according to an embodiment.
FIG. 10 is a view for describing a body shape selected on the basis of body information of a user according to an embodiment.
FIG. 11 is a view for describing a method of selecting a body shape according to an embodiment.
FIG. 12 is a view for describing a method of acquiring an ultrasonic image volume according to an embodiment.
FIG. 13 is a view for describing a method of inputting information on a scanning line according to an embodiment.
FIG. 14 is a view for describing a method of inputting information on a scanning line according to an embodiment.

### [Modes of the Invention]

The present specification describes the principles of the present invention and discloses embodiments such that the scope of the present invention may be clarified and those skilled in the art to which the present invention pertains may implement the present invention. The disclosed embodiments may be implemented in various forms.

Throughout the specification, like reference numerals refer to like elements. The present specification does not describe all components of embodiments, and common descriptions in the technical field to which the present invention pertains and redundant descriptions between the embodiments will be omitted. Terms such as "part" and "portion" used herein denote those that may be implemented by software or hardware, and according to embodiments, a plurality of parts or portions may be implemented by a single unit or element, or a single part or portion may include a plurality of units or elements. Hereinafter, an operation principle and the embodiments of the present invention will be described with reference to the accompanying drawings.

In the present specification, an "image" may include a medical image obtained by a medical imaging apparatus such as a magnetic resonance imaging (MRI) device, a computed tomography (CT) device, an ultrasound imaging device, and an X-ray imaging device.

In the present specification, an "object" is to be photographed and may include a person, an animal, or a part thereof. For example, the object may include a part (organ) of a human body, a phantom, or the like.

Throughout the specification, an "ultrasonic image" means an image of the object, which is processed based on an ultrasonic signal transmitted to the object and reflected from the object.

Hereinafter, the embodiments will be described in detail with reference to the accompanying drawings.

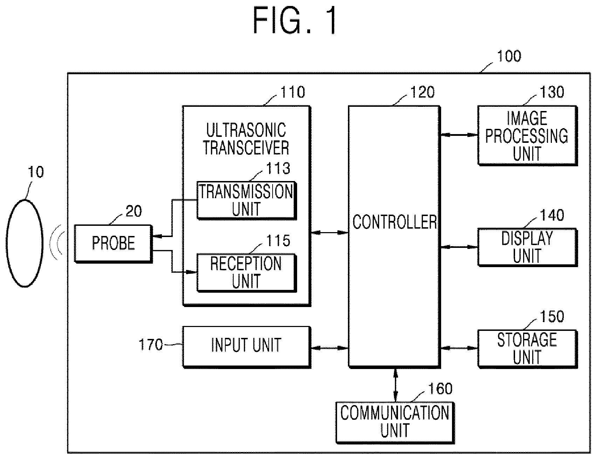

FIG. 1 is a block diagram illustrating a configuration of an ultrasound diagnosis apparatus 100 according to an embodiment. The ultrasound diagnosis apparatus 100 may include a probe 20, an ultrasonic transceiver 110, a controller 120, an image processing unit 130, a display unit 140, a storage unit 150, a communication unit 160, and an input unit 170.

The ultrasound diagnosis apparatus 100 may be implemented as a portable type as well as a cart type. Examples of a portable ultrasound diagnosis apparatus may include a smart phone, a laptop computer, a personal digital assistant (PDA), a tablet personal computer (PC), and the like including a probe and an application, but the present invention is not limited thereto.

The probe 20 may include a plurality of transducers. The plurality of transducers may transmit ultrasonic signals to an object 10 according to a transmission signal applied from a transmission unit 113. The plurality of transducers may receive ultrasonic signals reflected from the object 10 to form a reception signal. Further, the probe 20 may be implemented integrally with the ultrasound diagnosis apparatus 100 or may be implemented as a separate type in which the probe 20 is connected to the ultrasound diagnosis apparatus 100 in a wired or wireless manner. Further, the ultrasound diagnosis apparatus 100 may include one or more probes 20 according to an implementation form.

The controller 120 controls the transmission unit 113 to form a transmission signal to be applied to each of the plurality of transducers in consideration of the positions and focal points of the plurality of transducers included in the probe 20.

The controller 120 controls a reception unit 115 to convert a reception signal received from the probe 20 in an analog-to-digital conversion manner and to sum the digitally converted reception signal in consideration of the positions and focal points of the plurality of transducers, thereby generating ultrasonic data.

The image processing unit 130 generates an ultrasonic image using the ultrasonic data generated by the ultrasonic reception unit 115.

The display unit 140 may display the generated ultrasonic image and various pieces of information processed by the ultrasound diagnosis apparatus 100. The ultrasound diagnosis apparatus 100 may include one or more display units 140 according to an implementation form. Further, the display unit 140 may be implemented as a touch screen in combination with a touch panel.

The controller 120 may control the overall operation of the ultrasound diagnosis apparatus 100 and a signal flow between internal components of the ultrasound diagnosis apparatus 100. The controller 120 may include a memory that stores a program or data for performing a function of the ultrasound diagnosis apparatus 100 and a processor that processes the program or data. Further, the controller 120 may control the operation of the ultrasonic diagnosis device 100 by receiving a control signal from the input unit 170 or an external device.

The ultrasound diagnosis apparatus 100 may include the communication unit 160 and may be connected, through the communication unit 160, to an external device (for example, a server, a medical device, a portable device (a smart phone, a tablet PC, a wearable device, and the like)).

The communication unit 160 may include one or more components enabling communication with the external device and may include, for example, at least one of a short-range communication module, a wired communication module, and a wireless communication module.

The communication unit 160 may receive a control signal and data from the external device and transmit the received control signal to the controller 120 so that the controller 120 may control the ultrasound diagnosis apparatus 100 in response to the received control signal.

Alternatively, the controller 120 may transmit a control signal to the external device through the communication unit 160 so that the external device may be controlled in response to the control signal of the controller 120.

For example, the external device may process data of the external device in response to the control signal of the controller received through the communication unit.

A program capable of controlling the ultrasound diagnosis apparatus 100 may be installed in the external device, and the program may include instructions for performing some or all of the operations of the controller 120.

The program may be previously installed in the external device or may be installed by a user of the external device by downloading the program from a server that provides applications. The server that provides applications may include a recording medium in which the corresponding program is stored.

The storage unit 150 may store various types of data or programs for driving and controlling the ultrasound diagnosis apparatus 100, input/output ultrasonic data, acquired ultrasonic images, and the like.

The input unit 170 may receive a user's input for controlling the ultrasound diagnosis apparatus 100. Although the user's input may include, for example, input obtained by manipulating a button, a keypad, a mouse, a trackball, a jog switch, a knob, and the like, input obtained by touching a touch panel or a touch screen, voice input, motion input, biometric information input (for example, iris recognition, fingerprint recognition, and the like), and the like, the present invention is not limited thereto.

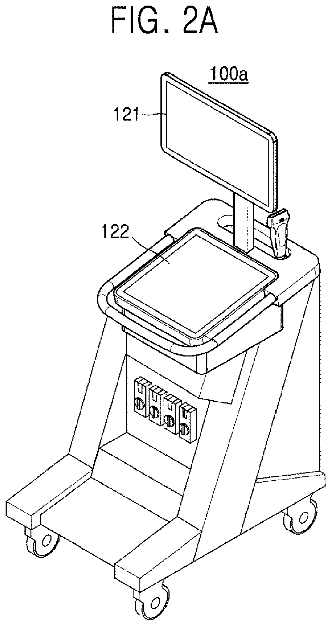

Examples of the ultrasound diagnosis apparatus 100 according to the embodiment will be described through FIGS. 2A to 2C.

FIGS. 2A to 2C are views illustrating ultrasound diagnosis apparatuses according to an embodiment.

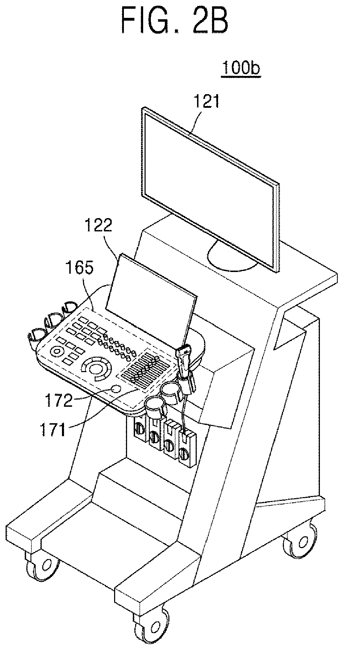

Referring to FIGS. 2A and 2B, ultrasound diagnosis apparatuses 100a and 100b may each include a main display unit 121 and a sub display unit 122. One of the main display unit 121 and the sub display unit 122 may be implemented as a touch screen. The main display unit 121 and the sub display unit 122 may display the ultrasonic image or various pieces of information processed by the ultrasound diagnosis apparatuses 100a and 100b. Further, the main display unit 121 and the sub display unit 122 may be implemented as a touch screen and provide a graphical user interface (GUI) to receive data for controlling the ultrasound diagnosis apparatuses 100a and 100b from a user. For example, the main display unit 121 may display the ultrasonic image, and the sub display unit 122 may display a control panel for controlling the ultrasonic image in the form of the GUI. The sub display unit 122 may receive data for controlling the displaying of the image through the control panel displayed in the form of the GUI. The ultrasound diagnosis apparatuses 100a and 100b may control, using input control data, the displaying of the ultrasonic image displayed on the main display unit 121.

Referring to FIG. 2B, the ultrasound diagnosis apparatus 100b may further include a control panel 165 in addition to the main display unit 121 and the sub display unit 122. The control panel 165 may include a button, a trackball, a jog switch, a knob, and the like, and may receive data for controlling the ultrasound diagnosis apparatus 100b from the user. For example, the control panel 165 may include a time gain compensation (TGC) button 171, a freeze button 172, and the like. The TGC button 171 is a button for setting a TGC value for each depth of the ultrasonic image. Further, when detecting the input of the freeze button 172 while scanning the ultrasonic image, the ultrasound diagnosis apparatus 100b may maintain a state in which a frame image at a corresponding time point is displayed.

Meanwhile, inputs of the button, the trackball, the jog switch, the knob, and the like included in the control panel 165 may be provided to the GUI in the main display unit 121 or the sub display unit 122.

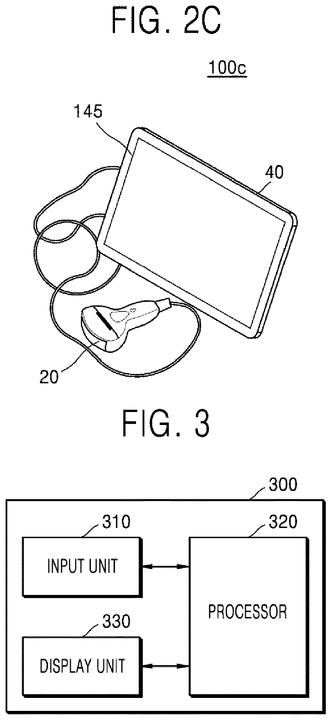

Referring to FIG. 2C, the ultrasound diagnosis apparatus 100c may be implemented as a portable type. Examples of a portable ultrasound diagnosis apparatus 100c may include a smart phone, a laptop computer, a PDA, a tablet PC, and the like including a probe and an application, but the present invention is not limited thereto.

The ultrasound diagnosis apparatus 100c may include the probe 20 and a main body 40, and the probe 20 may be connected to one side of the main body 40 in a wired or wireless manner. The main body 40 may include a touch screen 145. The touch screen 145 may display the ultrasonic image, various pieces of information processed by the ultrasound diagnosis apparatus, the GUI, and the like.

FIG. 3 is a block diagram illustrating a configuration of an ultrasound diagnosis apparatus according to an embodiment.

An ultrasound diagnosis apparatus 300 according to an embodiment includes an input unit 310, a processor 320, and a display unit 330. The ultrasound diagnosis apparatus 300 may correspond to the ultrasound diagnosis apparatus 100 of FIG. 1. In addition, the ultrasound diagnosis apparatus 300 may be implemented in the form illustrated in the ultrasound diagnosis apparatuses 100a, 100b, and 100c of FIG. 2. In an embodiment, the input unit 310 of FIG. 3 may include the input unit 170 of FIG. 1. Further, the processor 320 may correspond to the controller 120 and the image processor 130 of FIG. 1. The processor 320 may include one or a plurality of processors. The display unit 330 may correspond to the display unit 140 of FIG. 1.

According to an embodiment, the ultrasound diagnosis apparatus 300 may include fewer components than those shown in FIG. 3 or may further include other additional components. For example, the ultrasound diagnosis apparatus 300 may receive a user input from a separate device instead of including the input unit 310.

According to an embodiment, the ultrasound diagnosis apparatus 300 may include a probe configured to transmit ultrasonic signals to an object and detect ultrasonic echo signals. In an embodiment, the probe may transmit ultrasonic signals to the object while moving on the basis of a scanning line and receive ultrasonic echo signals reflected from the object. In this case, the scanning line may refer to a moving line along which the probe moves from a scan start position to a scan end position in order to scan the object. The scan start position may be a position of the probe at the starting of acquiring the ultrasonic data for the object. The scan end position may be a position of the probe at the ending of acquiring the ultrasonic data for the object.

In an embodiment, the probe may be a freehand type probe. The probe may be a linear probe or a two-dimensional matrix array type probe, but in the present embodiment, the type of probe is not limited to the above-described example.

The processor 320 may acquire ultrasonic data for the object from the ultrasonic echo signals. According to an embodiment, the processor 320 may acquire brightness (B) mode image data from the ultrasonic echo signals. Alternatively, the processor 320 may acquire, from the ultrasonic echo signals, ultrasonic image data including at least one of spectral Doppler image data, color Doppler image data, elasticity image data, and motion (M) mode image data, but the types of ultrasonic image data that are to be acquired by the processor 320 are not limited thereto. Meanwhile, the color Doppler image data may include at least one of blood flow Doppler image data and tissue Doppler image data.

In an embodiment, in an embodiment, the ultrasonic data may be cross-sectional data of the object. The cross-sectional data of the object may include data based on a cross section of the object or a predetermined volume centered on the cross section. For example, the cross-sectional data of the object may be two-dimensional image data showing a cross section of the object or three-dimensional image data on a predetermined volume centered on the cross section of the object.

The processor 320 may control the display unit 330 to display a body shape of a user. In an embodiment, the body shape of the user may be provided in the type of at least one of a front view, a rear view, a cross-sectional view, and a three-dimensional image of the body of the user, or an outline thereof. In addition, the body shape of the user may be provided for a whole body of the user or a part of the body of the user.

The processor 320 may acquire information on the scanning line through the input unit 310. In an embodiment, the information on the scanning line may be acquired in the form of information indicating a scan start position and a scan end position on the displayed body shape. As an example, the input unit 310 may provide a user interface for inputting a point (hereinafter, referred to as a start point) indicating the scan start position on the displayed body shape. In addition, the input unit 310 may provide a user interface for inputting a point (hereinafter, referred to as an end point) indicating the scan end position on the displayed body shape.

The processor 320 may approximate the scanning line in the form of a line connecting the above-described scan start position and scan end position. As an example, the scanning line may be approximated in the form of a straight line connecting the above-described scan start position and scan end position at the shortest distance. Alternatively, the scanning line may be approximated in the form of a free curve connecting the above-described scan start position and scan end position on the basis of the body shape. For example, the scanning line may be approximated in the form of a free curve connecting the above-described scan start position and scan end position along a curve of the body shape.

In an embodiment, the information on the scanning line may be acquired in the form of any line representing the scanning line on the displayed body shape. As an example, the input unit 310 may provide an interface for drawing a free curve representing the scanning line on the displayed body shape. The processor 320 may determine a position at which the input of the above-described free curve starts as a start point, and determine a position at which the input ends as an end point.

The processor 320 may acquire the ultrasonic image volume from the ultrasonic data on the basis of the body shape and the information on the scanning line. In an embodiment, the ultrasonic image volume may be a volume representing an image of a part (hereinafter, referred to as a measured part of the object) of the object, which is scanned corresponding to the scanning line.

In order to acquire the ultrasonic image volume with a high degree of realization, it is important to determine an entire length of the measured part of the object. In an embodiment, the processor 320 may calculate a length of the measured part of the object on the basis of the body shape and the information on the scanning line, and acquire the ultrasonic image volume from the ultrasonic data on the basis of the calculated length. For example, the ultrasound diagnosis apparatus may calculate a length of the scanning line using a scale of the body shape. The ultrasound diagnosis apparatus may approximate the calculated length of the scanning line as the length of the measured part of the object.

The processor 320 may be configured as a hardware unit including a memory for storing at least one of a program, an algorithm, and application data for acquiring the ultrasonic image volume from the ultrasonic data on the basis of the body shape and the information on the scanning line, and a processor for processing the program, algorithm, or application data stored in the memory. For example, the processor 320 may be configured as a processor including at least one of a central processing unit, a microprocessor, and a graphic processing unit. In this case, the memory and the processor may be formed as a single chip, but the present invention is not limited thereto.

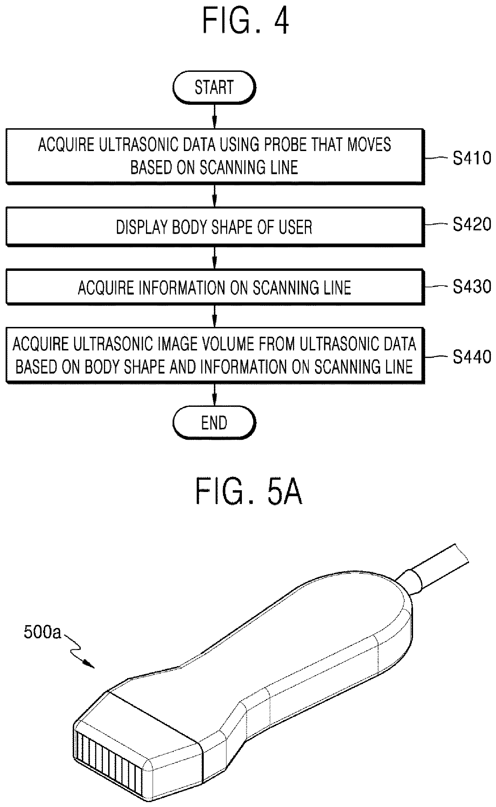

FIG. 4 is a flowchart illustrating a method of displaying an ultrasonic image according to an embodiment.

In step S410, ultrasonic data may be acquired. The ultrasonic data may be acquired using a probe that moves on the basis of a scanning line. In an embodiment, the scanning line may refer to a moving line along which the probe moves from a scan start position to a scan end position in order to scan an object.

In an embodiment, the probe may continuously acquire the ultrasonic data while moving along the scanning line. Alternatively, the probe may acquire the ultrasonic data at equal intervals with a period of predetermined time. Alternatively, the probe may acquire the ultrasonic data at a variable time interval. The method of acquiring the ultrasonic data by the probe is not limited to the above-described example.

In an embodiment, the ultrasonic data may be cross-sectional data of the object. The cross-sectional data of the object may include data based on a cross section of the object or a predetermined volume centered on the cross section. For example, the cross-sectional data of the object may include a two-dimensional image showing a cross section of the object or a three-dimensional image for a predetermined volume centered on the cross section of the object. The cross-sectional data of the object may be determined according to the type of probe.

In step S420, a body shape of a user may be displayed. In an embodiment, the body shape of the user may be provided in the form of at least one of a front view, a rear view, a cross-sectional view, and a three-dimensional image of the body of the user, or an outline thereof. In addition, the body shape of the user may be provided for a whole body of the user or a part of the body of the user.

In an embodiment, a plurality of symbols respectively corresponding to a plurality of parts of the body of the user may be displayed. For example, each of the symbols may be an icon indicating a corresponding body part, or a text indicating a name of the body part, but is not limited to the above-described example. A signal for selecting one of the plurality of parts of the body described above may be acquired through a user interface. In an embodiment, the signal for selecting one part may be acquired in a manner that selects one of the plurality of symbols described above. In addition, a shape of the selected part among the plurality of parts of the body of the user may be provided as the body shape of the user described above.

In an embodiment, the body shape of the user may be displayed using body information of the user. The body information of the user may include height of the user. In addition, the body information of the user may include at least one of age, gender, and weight of the user.

In an embodiment, a scale of the displayed body shape of the user may be determined on the basis of the height of the user.

In an embodiment, the ultrasound diagnosis apparatus may store a library including a plurality of standard body shapes, or may acquire a body shape from a library stored outside. In an embodiment, the displayed body shape of the user may be determined from the plurality of standard body shapes stored in the library on the basis of the above-described body information.

In an embodiment, the body information of the user may include identification information of the user. The ultrasound diagnosis apparatus may display the previously stored body shape as the body shape of the user on the basis of the identification information of the user.

In an embodiment, the body information of the user may be acquired through a user interface. As an example, the user interface may provide a text field for inputting the height of the user. The ultrasound diagnosis apparatus may use a value acquired by using the text field as the height of the user.

In step S430, information on the scanning line may be acquired. In an embodiment, the information on the scanning line may be acquired using a user interface.

In an embodiment, the information on the scanning line may be acquired in the form of information indicating a scan start position and a scan end position on the displayed body shape. For example, the user interface may provide an interface for inputting a point (hereinafter, referred to as a start point) indicating the scan start position on the displayed body shape. In addition, the user interface may provide an interface for inputting a point (hereinafter, referred to as an end point) indicating the scan end position on the displayed body shape.

In an embodiment, the scanning line may be approximated in the form of a line connecting the above-described scan start position and scan end position. As an example, the scanning line may be approximated in the form of a straight line connecting the above-described scan start position and scan end position at the shortest distance. Alternatively, the scanning line may be approximated in the form of a free curve connecting the above-described scan start position and scan end position on the basis of the body shape. For example, the scanning line may be approximated in the form of a free curve connecting the above-described scan start position and scan end position along a curve of the body shape.

In the above-described embodiment, in order to approximate the scanning line, the method of acquiring information indicating the scan start position and the scan end position has been proposed, but in the present embodiment, scan position information acquired to approximate the scanning line is not limited to the scan start position and the scan end position. For example, the information on the scanning line may be acquired in the form of information indicating a plurality of positions, which include information indicating the scan start position and the scan end position, on the displayed body shape. That is, the user interface may provide an interface for inputting a plurality of points, including the start point and the end point, indicating a plurality of scan positions on the body shape. The user interface may provide an interface that aligns the plurality of points in the order of scan time-series. In this case, the scanning line may be approximated in the form of lines connecting the plurality of scan positions.

In another embodiment, the scanning line may be acquired in the form of any line representing the scanning line on the displayed body shape. As an example, the user interface may provide an interface for drawing a free curve representing the scanning line on the displayed body shape. The ultrasound diagnosis apparatus may determine a position at which the input of the above-described free curve starts as a start point, and determine a position at which the input ends as an end point.

In step S440, an ultrasonic image volume may be acquired from the ultrasonic data on the basis of the body shape and the information on the scanning line. In an embodiment, the ultrasonic image volume may be a volume representing an image of a part (hereinafter, referred to as a measured part of the object) of the object, which is scanned corresponding to the scanning line.

In an embodiment, the ultrasound diagnosis apparatus may calculate a length of the measured part of the object on the basis of the body shape and the information on the scanning line, and acquire the ultrasonic image volume from the ultrasonic data on the basis of the calculated length.

For example, the ultrasound diagnosis apparatus may calculate a length of the scanning line using a length of the line, which represents the scanning line, on the body shape and a scale of the body shape. The ultrasound diagnosis apparatus may approximate the calculated length of the scanning line as the length of the measured part of the object.

In an embodiment, it will be apparent to those skilled in the art that the above-described steps for displaying an ultrasonic image may not necessarily be sequential. For example, the information on the scanning line may be acquired prior to acquiring the ultrasonic data.

In an embodiment, the ultrasound diagnosis apparatus may generate and store a profile for the acquired ultrasonic image volume. The profile may include information on the scanning line and the body shape displayed to acquire the ultrasonic image volume. In addition, the profile may include identification information for identifying the acquired ultrasonic image volume and/or identification information for the user to be measured. Thereafter, the ultrasound diagnosis apparatus may provide a guideline for the scanning line on the basis of the stored profile when acquiring an ultrasonic image at the same position is requested for the same user.

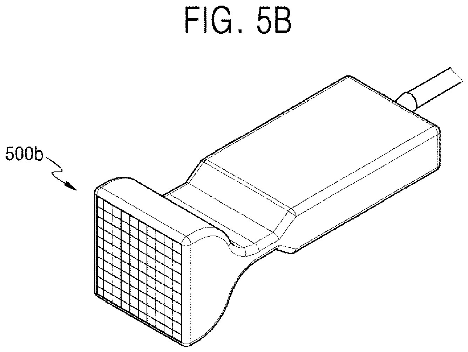

FIGS. 5A and 5B are views for describing a probe according to an embodiment.

Referring to FIGS. 5A and 5B, a probe 500a may be a freehand type probe. Referring to FIG. 5A, the probe 500a may be a linear probe. The probe 500a may include transducers arranged in the form of a straight line. The probe 500a may continuously acquire ultrasonic data of an object while moving along a scanning line from a scan start position to a scan end position.

Referring to FIG. 5B, a probe 500b may be a two-dimensional matrix array type probe. The probe 500b may include transducers arranged in a two-dimensional matrix form. The probe 500b may continuously acquire ultrasonic data for providing a three-dimensional (3D) ultrasound stereoscopic image of the object while moving along a scanning line from a scan start position to a scan end position. However, the technical features of the present invention are not limited to the types of probe described above, and the probe may be provided in various forms such as a phased array probe, a 3D matrix probe, and the like.

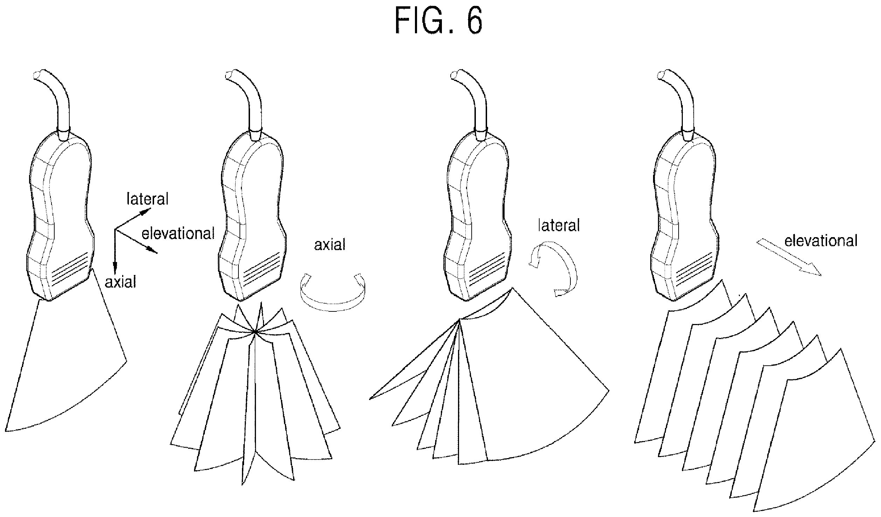

FIG. 6 is a view for describing the movement of the probe according to an embodiment.

Referring to FIG. 6, three directions forming a right angle to each other around the probe, that is, an axial direction A, a lateral direction L, and an elevation direction E may be defined. In an embodiment, a direction in which an ultrasonic signal is irradiated may be defined as the axial direction A, a direction in which transducers form a row may be defined as the lateral direction L, and a direction perpendicular to the axial direction A and the lateral direction L may be defined as the elevation direction E. The movement of the probe may be expressed based on the directions described above. In an embodiment, the scanning line may include all information on the three directions described above. However, this is merely exemplary, and the scanning line may be provided in other forms known in the art in addition to those illustrated, such as including only information on the movement of a center position of the probe or only some of the information on the three directions described above.

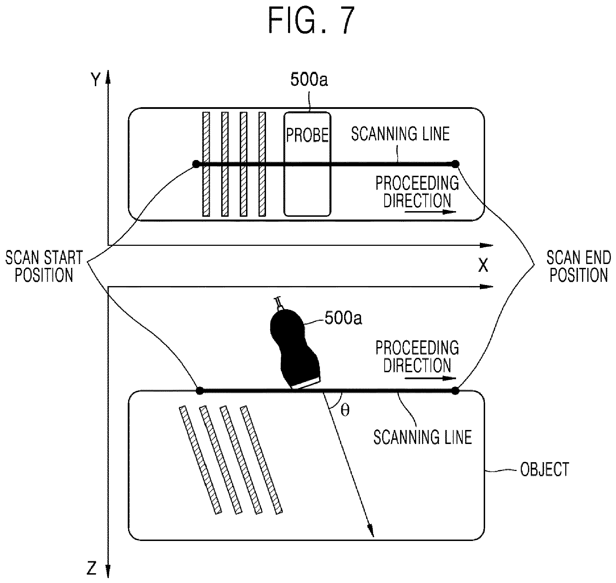

FIG. 7 is a view for describing a method of acquiring ultrasonic data of an object using the probe of FIG. 5A. Referring to FIG. 7, the probe 500a may acquire ultrasonic data for the object while moving in a proceeding direction along a scanning line from a scan start position to a scan end position. The scan start position may be a position of the probe 500a at the starting of acquiring the ultrasonic data for the object. The scan end position may be a position of the probe 500a at the ending of acquiring the ultrasonic data for the object.

In an embodiment, the probe 500a may continuously acquire the ultrasonic data. Alternatively, the probe 500a may acquire the ultrasonic data at equal intervals with a period of predetermined time. Alternatively, the probe 500a may acquire the ultrasonic data at a variable time interval.

In an embodiment, the ultrasonic data may be cross-sectional data of the object. The cross-sectional data of the object may be data based on a cross section of the object. For example, the cross-sectional data of the object may be a two-dimensional image showing a cross section of an object.

In an embodiment, the probe 500a may transmit a signal for acquiring ultrasonic data toward the object at an angle of a transmission angle θ. In an embodiment, the ultrasonic data acquired by the probe 500a may be data based on a cross section of the object in a direction of the transmission angle θ or a predetermined volume centered on the cross section. In an embodiment, the probe 500a may include an additional component for measuring the transmission angle θ. Alternatively, the probe 500a may move at a fixed angle to transmit a signal at the predetermined transmission angle θ.

The ultrasound diagnosis apparatus may acquire an ultrasonic image volume on the basis of one or more pieces of cross-sectional data of the object.

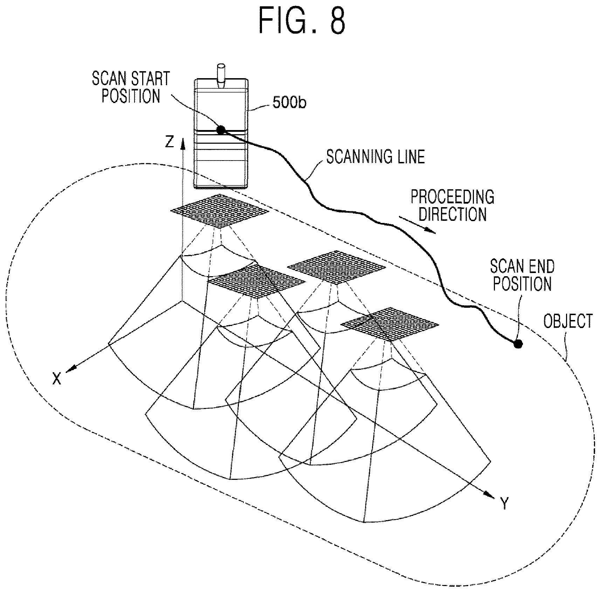

FIG. 8 is a view for describing a method of acquiring ultrasonic data of an object using the probe of FIG. 5B. Referring to FIG. 8, the probe 500b may acquire ultrasonic data for the object while moving in a proceeding direction along a scanning line from a scan start position to a scan end position. In an embodiment, the probe 500b may continuously acquire the ultrasonic data. Alternatively, the probe 500b may acquire the ultrasonic data at equal intervals with a period of predetermined time. Alternatively, the probe 500b may acquire the ultrasonic data at a variable time interval.

In an embodiment, the ultrasonic data may be cross-sectional data of the object. The cross-sectional data of the object may include data based on a predetermined volume centered on a cross section of the object. For example, the cross-sectional data of the object may be a three-dimensional image showing a predetermined volume centered on the cross section of the object.

The ultrasound diagnosis apparatus may acquire an ultrasonic image volume on the basis of one or more pieces of cross-sectional data of the object.

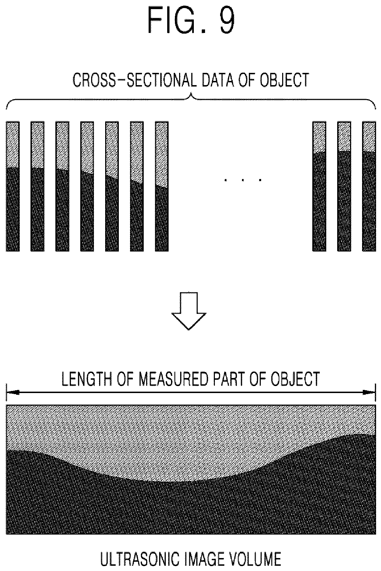

FIG. 9 is a view for describing a method of acquiring an ultrasonic image volume on the basis of ultrasonic data according to an embodiment. Referring to FIG. 9, the ultrasound diagnosis apparatus may acquire an ultrasonic image volume of a part of an object corresponding to a scanning line.

In FIG. 9, the ultrasonic data may be cross-sectional data of the object. In the following description, the cross-sectional data of the object may include data based on a cross section of the object or a predetermined volume centered on the cross section. The probe may acquire one or more pieces of cross-sectional data of the object for the object while moving in a proceeding direction along a scanning line from a scan start position to a scan end position. The ultrasound diagnosis apparatus may acquire the ultrasonic image volume on the basis of one or more pieces of cross-sectional data of the object.

The ultrasonic image volume is a volume representing an image of a part (hereinafter, referred to as a measured part of the object) of the object, which is scanned corresponding to the scanning line. In an embodiment, the ultrasound diagnosis apparatus may acquire the ultrasonic image volume by matching one or more pieces of cross-sectional data of the object.

In order to acquire the ultrasonic image volume with high accuracy, it is important to determine an entire length of the measured part of the object. In an embodiment, the ultrasound diagnosis apparatus may calculate a length of the measured part of the object on the basis of a body shape and the scanning line, and acquire the ultrasonic image volume from the cross-sectional data of the object on the basis of the calculated length.

Meanwhile, the ultrasound diagnosis apparatus may acquire the ultrasonic image volume from the cross-sectional data of the object by further using the shape of the scanning line. For example, when the scanning line is a curved line, the ultrasound diagnosis apparatus may acquire the ultrasonic image volume formed in a curved shape on the basis of the scanning line.

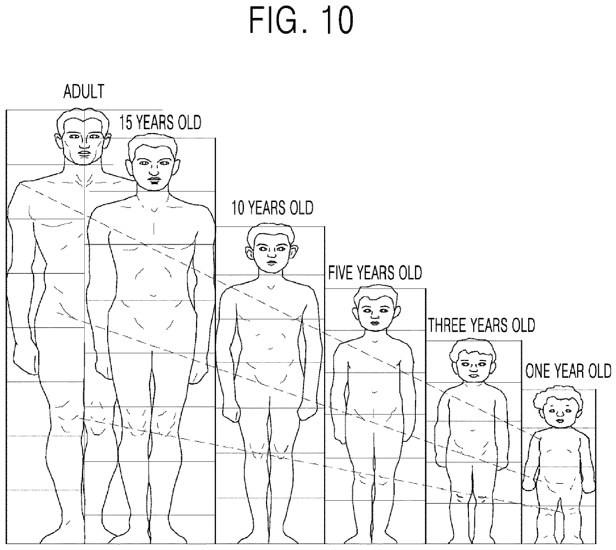

FIG. 10 is a view for describing a body shape selected on the basis of body information of a user according to an embodiment. In FIG. 10, as an example, the body information of the user is illustrated as including age, gender, and height of the user. However, it will be fully understood by those skilled in the art that the body information of the present embodiment is not limited to the example described above.

Referring to FIG. 10, according to the gender, age, and height of the user, a life size and form of the body shape of the user may be predicted differently. In an embodiment, the ultrasound diagnosis apparatus may select a body shape to be displayed, among a plurality of standard body shapes stored in a library, on the basis of the body information of the user.

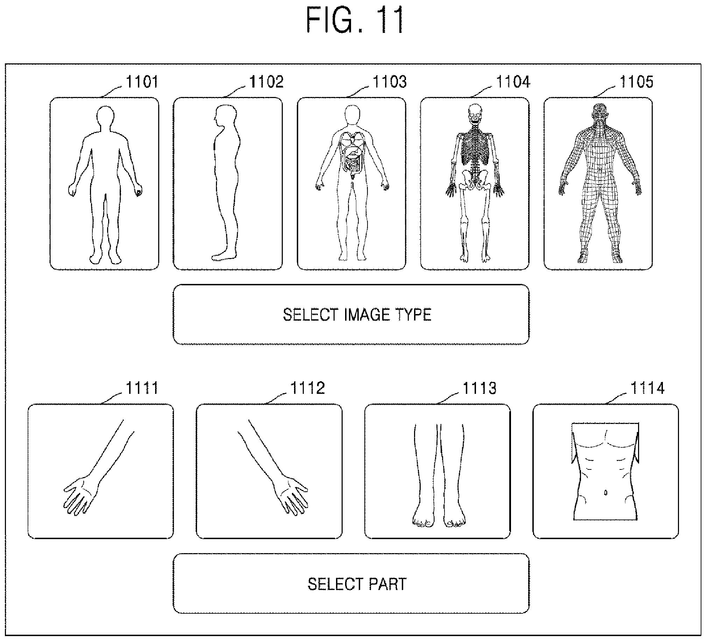

FIG. 11 is a view for describing a method of selecting a body shape according to an embodiment. Referring to FIG. 11, the ultrasound diagnosis apparatus may provide a user interface for selecting the type of body shape, and a user interface for selecting a part of a body of a user as the body shape. Although two user interfaces are displayed on one display unit in FIG. 11, it will be fully understood by those skilled in the art that the ultrasound diagnosis apparatus may provide the user interfaces in various ways, such as providing each user interface individually or sequentially.

Referring to FIG. 11, the ultrasound diagnosis apparatus may display a plurality of symbols 1101, 1102, 1103, 1104, and 1105 corresponding to a plurality of types of body shape. In FIG. 11, each of the plurality of symbols is illustrated as a symbol 1101 corresponding to a front view of the body, a symbol 1102 corresponding to a side view of the body, a symbol 1103 corresponding to a skeletal view of the body, a symbol 1104 corresponding to the anatomical view of the body, and a symbol 1105 corresponding to a 3D image of the body. However, in the present embodiment, the types of body shape are not limited to the above-described examples, and may be provided in various forms such as a front view, a rear view, a cross-sectional view, a three-dimensional image, and the like of the body.

Referring to FIG. 11, the ultrasound diagnosis apparatus may display a plurality of symbols 1111, 1112, 1113, and 1114 corresponding to a plurality of parts of the body of the user. In FIG. 11, each of the plurality of symbols is illustrated as a symbol 1111 corresponding to a left arm, a symbol 1112 corresponding to a right arm, a symbol 1113 corresponding to legs, and a symbol 1114 corresponding to a torso. However, in the present embodiment, the parts of the body are not limited to the examples described above, and may be provided in various forms such as an upper body, a lower body, a head part, and the like.

Meanwhile, the ultrasound diagnosis apparatus may provide a user interface capable of receiving a signal for selecting the type of body shape on the basis of the plurality of displayed symbols 1101, 1102, 1103, 1104, and 1105. As an example, the signal for selecting the type of body shape may be input in a manner that selects one of the symbols 1101, 1102, 1103, 1104, and 1105.

In addition, the ultrasound diagnosis apparatus may provide a user interface capable of receiving a signal for selecting one of a plurality of body parts on the basis of the plurality of displayed symbols 1111, 1112, 1113, and 1114. As an example, the signal for selecting the body part may be input in a manner that selects one of the symbols 1111, 1112, 1113, and 1114.

In an embodiment, the ultrasound diagnosis apparatus may display the selected body part as a selected body shape type.

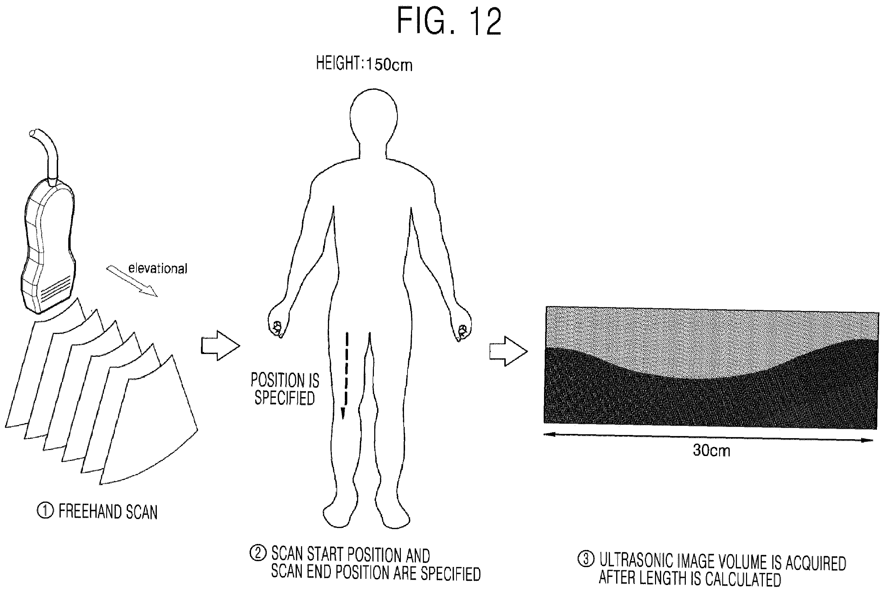

FIG. 12 is a view for describing a method of acquiring an ultrasonic image volume according to an embodiment. Referring to FIG. 12, ultrasonic data for an object may be acquired using a movable probe, for example, a freehand type probe.

Meanwhile, the ultrasound diagnosis apparatus may display a body shape of a user. Although the body shape of the user is illustrated in the form of a front view of a whole body in FIG. 12, it will be fully understood that the types of body shape and the body parts displayed by the ultrasound diagnosis apparatus are not limited thereto.

The ultrasound diagnosis apparatus may acquire information on a scanning line using a user interface. In FIG. 12, as an example, a user interface for inputting a point (hereinafter, referred to as a start point) indicating a scan start position on the displayed body shape and a point indicating a scan end position on the body shape (hereinafter, referred to as an end point) is illustrated. The ultrasound diagnosis apparatus may approximate the scanning line using the acquired information on the scanning line.

The ultrasound diagnosis apparatus may acquire the ultrasonic image volume from the ultrasonic data on the basis of the body shape and the information on the scanning line. For example, the ultrasound diagnosis apparatus may calculate a length of the scanning line using a length of the line, which represents the scanning line, on the body shape and a scale of the body shape. The ultrasound diagnosis apparatus may approximate the calculated length of the scanning line as a length of a measured part of the object. As an example, when a user's height is 150 cm and the length of the line representing the scanning line on the body shape is 1/5 of a length of the body shape, a result that the length of the scanning line is approximated to be 30 cm is illustrated in FIG. 12. The ultrasound diagnosis apparatus may acquire the ultrasonic image volume on the basis of the calculated length.

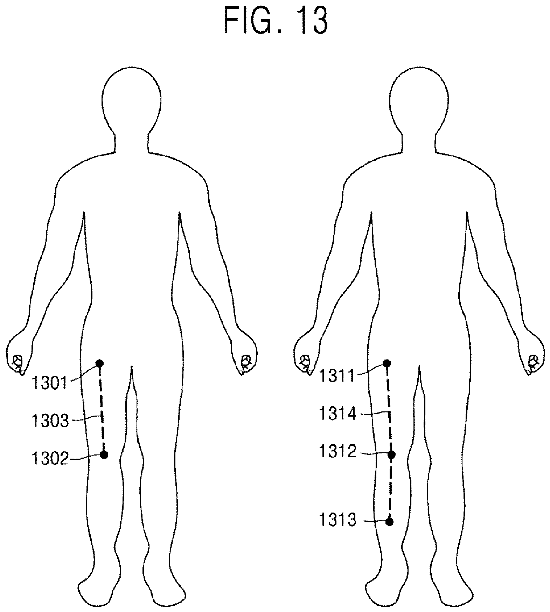

FIG. 13 is a view for describing a method of inputting information on a scanning line according to an embodiment. Referring to FIG. 13, the information on the scanning line may be input in a manner of inputting a position of a point indicating a scan position on a displayed body shape.

In an embodiment, a user interface may provide an interface for inputting a start point 1301, which is a point representing a scan start position on the displayed body shape, and an end point 1302, which is a point indicating a scan end position on the displayed body shape. In this case, the scanning line may be approximated in the form of a line 1303 connecting the above-described scan start position and scan end position. In FIG. 13, as an example, the line is approximated in the form of a straight line connecting the scan start position and the scan end position at the shortest distance.

Meanwhile, the information on the scanning line may be acquired in the form of information indicating a plurality of positions, which include information indicating the scan start position and the scan end position, on the displayed body shape. That is, the user interface may provide an interface for inputting a plurality of points 1311 to 1313, which include a start point 1311 and an end point 1313, indicating a plurality of scan positions on the body shape. The user interface may provide an interface that aligns the plurality of points 1311 to 1313 in the order of scan time-series. In this case, the scanning line may be approximated in the form of a line 1314 connecting the plurality of scan positions. In FIG. 13, as an example, the line is approximated in the form of a polygonal line connecting between the scan start position, at least one scan intermediate position, and the scan end position at the shortest distance.

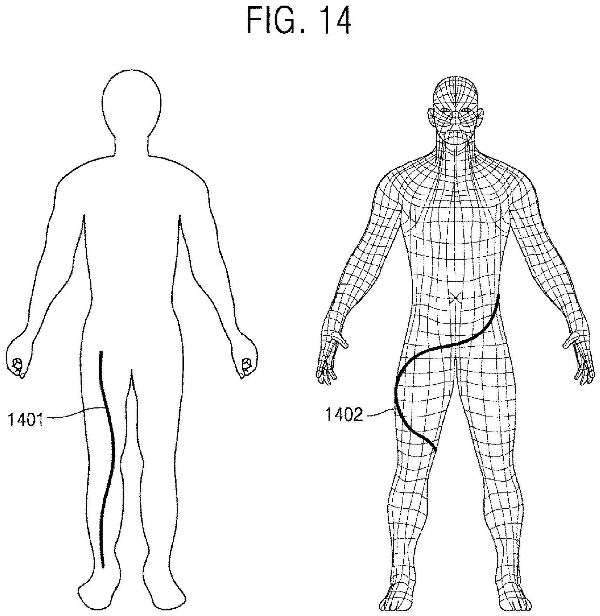

FIG. 14 is a view for describing a method of inputting information on a scanning line according to an embodiment. Referring to FIG. 14, the information on the scanning line may be input in the form of any line representing a scanning line on a displayed body shape.

In an embodiment, a user interface may provide an interface for drawing a free curve 1401 representing the scanning line on the displayed body shape. The ultrasound diagnosis apparatus may determine a position at which the input of the above-described free curve 1401 starts as a start point, and determine a position at which the input ends as an end point.

Meanwhile, when the body shape is a three-dimensional image, the user interface may provide an interface for drawing a three-dimensional free curve 1402 representing a scanning line on the displayed body shape. The ultrasound diagnosis apparatus may determine a position at which the input of the above-described free curve 1402 starts as a start point, and determine a position at which the input ends as an end point.

Meanwhile, the disclosed embodiments may be implemented in the form of a computer-readable recording medium storing instructions and data executable by a computer. The instructions may be stored in the form of program codes, and when executed by a processor, generate a predetermined program module to perform a predetermined operation. Further, when being executed by the processor, the instructions may perform predetermined operations of the disclosed embodiments.

## Claims

1. A method of displaying an ultrasonic image, the method comprising steps of:
acquiring ultrasonic data using a probe that moves on the basis of a scanning line;
displaying a body shape of a user;
acquiring information on the scanning line through a user interface; and
acquiring an ultrasonic image volume from the ultrasonic data on the basis of the body shape and the information on the scanning line.

2. The method of claim 1, further comprising a step of acquiring a height of the user through the user interface,
wherein the step of acquiring the ultrasonic image volume from the ultrasonic data is a step of acquiring an ultrasonic data volume from the ultrasonic data on the basis of the height, the body shape, and the information on the scanning line.

3. The method of claim 1, wherein the body shape is a shape that illustrates a part of a body of the user.

4. The method of claim 3, wherein the step of displaying the body shape of the user includes steps of:
displaying a plurality of symbols corresponding to a plurality of parts of the body of the user;
acquiring a signal for selecting one of the plurality of parts through the user interface; and
displaying the selected part based on the signal as the body shape.

5. The method of claim 1, wherein the information on the scanning line is acquired in a form of information indicating a start point and an end point of the scanning line on the displayed body shape.

6. The method of claim 1, wherein the information on the scanning line is acquired in a form of a line indicating the scanning line on the displayed body shape.

7. The method of claim 1, wherein the body shape is provided in at least one type of a front view, a rear view, a cross-sectional view, and a three-dimensional image of the body of the user.

8. An ultrasound diagnosis apparatus comprising:
a display unit;
a user interface;
a memory for storing one or more instructions; and
a processor for executing the one or more instructions to acquire ultrasonic data using a probe that moves on the basis of a scanning line, control the display unit to display a body shape of a user, control the user interface to acquire information on the scanning line, and acquire, on the basis of the body shape and the information on the scanning line, an ultrasonic image volume from the ultrasonic data.

9. The ultrasound diagnosis apparatus of claim 8, wherein the processor controls the user interface to acquire a height of the user, and acquires an ultrasonic data volume from the ultrasonic data on the basis of the height, the body shape, and the information on the scanning line.

10. The ultrasound diagnosis apparatus of claim 8, wherein the body shape is a shape that illustrates a part of a body of the user.

11. The ultrasound diagnosis apparatus of claim 10, wherein the processor controls the display unit to display a plurality of symbols corresponding to a plurality of parts of the body of the user, acquires a signal for selecting one of the plurality of parts through the user interface, and controls the display unit to display the selected part based on the signal as the body shape.

12. The ultrasound diagnosis apparatus of claim 8, wherein the information on the scanning line is acquired in a form of information indicating a start point and an end point of the scanning line on the displayed body shape.

13. The ultrasound diagnosis apparatus of claim 8, wherein the information on the scanning line is acquired in a form of a line indicating the scanning line on the displayed body shape.

14. The ultrasound diagnosis apparatus of claim 8, wherein the body shape is provided in at least one type of a front view, a rear view, a cross-sectional view, and a three-dimensional image of a body of the user.

15. A computer-readable recording medium configured to store a computer program code, which performs a method of displaying an ultrasonic image when being read and executed by a processor, wherein the method of displaying an ultrasonic image comprises steps of:
acquiring ultrasonic data using a probe that moves on the basis of a scanning line;
displaying a body shape of a user;
acquiring information on the scanning line through a user interface; and
acquiring an ultrasonic image volume from the ultrasonic data on the basis of the body shape and the information on the scanning line.
